# EUROPÄISCHE PATENTSCHRIFT

(11) **EP 0 892 794 B1**
(45) Veröffentlichungstag und Bekanntmachung des Hinweises auf die Patenterteilung: **06.02.2002**
(21) Anmeldenummer: 97916380.5
(22) Anmeldetag: 22.03.1997
(51) Int. Cl.: C07D 401/04, A61K 31/445

(54) **ACYLIERTE N-HYDROXYMETHYL THALIDOMID-PRODRUGS MIT IMMUNOMODULATORISCHER WIRKUNG**
ACYLATED N-HYDROXY METHYL THALIDOMIDE PRODRUGS WITH IMMUNOMODULATOR ACTION
PROMEDICAMENTS ACYLES DE LA N-HYDROXYMETHYLTHALIDOMIDE A EFFET IMMUNOMODULATEUR

(30) Priorität: 09.04.1996 DE 19613976
(43) Veröffentlichungstag der Anmeldung: 27.01.1999
(73) Patentinhaber: Grünenthal GmbH, 52078 Aachen (DE)
(72) Erfinder: SCHNEIDER, Johannes, D-52223 Stolberg (DE); WINTER, Werner, D-52078 Aachen (DE); WNENDT, Stephan, D-52076 Aachen (DE); ZWINGENBERGER, Kai, D-52076 Aachen (DE); EGER, Kurt, D-72072 Tübingen (DE); AKERMANN, Michaela, D-72184 Eutingen (DE)
(86) Internationale Anmeldenummer: EP9701475
(87) Internationale Veröffentlichungsnummer: WO9737988

(56) Entgegenhaltungen:
- WO-A-92/14455
- DE-A- 4 211 812
- ARCH. PHARM. (WEINHEIM) (1969), 302(3), 188-96 CODEN: APBDAJ, 1969, XP000675199 WERNER, WALTER ET AL: "Potential cytostatics from aminomethylation of NH-acid hypnotics"
- ACTA PHYS. CHEM. (1966), 12(3-4), 143-50 CODEN: AUSHAF, 1966, XP000675372 KOVACS, KALMAN ET AL: "Synthesis of glutamine and pyroglutamylglutamine derivatives substituted in carboxamide nitrogen"
- J. INDIAN CHEM. SOC. (1976), 53(11), 1122-5 CODEN: JICSAH, 1976, XP000675187 DE, A. U. ET AL: "Possible antineoplastic agents. III. Synthesis of 6-alkyl-2-[4'-methoxyphthalimido] and 6-alkyl-3-[3'-4'- dimethoxyphenyl]glutarimides"
- INDIAN J. CHEM., SECT. B (1978), 16B(6), 510-12 CODEN: IJSBDB;ISSN: 0376-4699, 1978, XP000675183 DE, A. U. ET AL: "Possible antineoplastic agents: part IV. Synthesis and antineoplastic potency of N-substituted.alpha.-(4,5- dimethoxyphthalimido)glutarimides and N-substituted.beta.-(4-bromophenyl)glutari mides"

## Beschreibung

Die Erfindung betrifft Thalidomid-Prodrugs, Verfahren zu deren Herstellung sowie die Verwendung derselben als Arzneimittelwirkstoff.

Die überschießende Bildung des Zytokins TNF-α (Tumornekrosefaktor α) spielt eine zentrale Rolle in der Pathogenese des Graft-versus-Host-Syndroms, der Multiplen Sklerose, der Transplantatabstoßung, aphthöser Stomatitis, Erythema nodosum leprosum, Morbus Boeck, rheumatoider Arthritis und einer Reihe weiterer Erkrankungen, die mit entzündlichen Erscheinungen einhergehen. Ein Ansatz für die Therapie dieser Erkrankungen besteht in der gezielten Unterdrückung der TNF-α-Freisetzung durch Gabe immunmodulierender Wirkstoffe, wie zum Beispiel Dexamethason, Pentoxifyllin oder Thalidomid.

Thalidomid hat sich in der Behandlung der aphthosen Stomatitis im Vergleich zu den klassischen Immunsuppressiva als überlegen herausgestellt. Weitere Beispiele von Erkrankungen, in denen Thalidomid eine gute Wirksamkeit zeigte, ohne zu einer generellen Immunsuppression zu führen, sind kutaner Lupus erythematodes, Pyoderma gangränosum und orogenitale Ulcera bei Morbus Behçet sowie histologisch von aphthösen Ulzera nicht abweichende Ulzerationen bei HIV-Infizierten, in denen - anders als bei der Mehrzahl der HIV-assoziierten mukokutanen Läsionen - keine mikrobiellen Erreger nachgewiesen werden können. Diese auch als Major-Aphthen zu bezeichnenden Läsionen können im Unterschied zur Stomatitis aphthosa im gesamten Verdauungstrakt auftreten und bei Lokalisation im Rachenraum oder der Speiseröhre durch ihre Schmerz verursachende Wirkung die Nahrungsaufnahme, aber auch die Einnahme oraler Medikation, erschweren. Als pathogenetische Faktoren gelten endogene Mediatoren mit Wirkungen auf das Endothel und zirkulierende Leukozyten. Unter dem Einfluß von lokal gebildetem TNF-α und anderer Zytokine nimmt fokal die Adhäsivität des Endothels gegenüber Leukozyten zu, was maßgeblich zur Ausbildung venöser Vaskulitiden beiträgt. Substanzen, die wie Thalidomid diese Veränderung des Endothels unterdrücken, ohne zugleich die spezifische zelluläre Immunabwehr zu blockieren, können einen bedeutenden Fortschritt für die Therapie darstellen.

Für schwere Fälle pharyngealer oder ösophagealer Ulzera, bei denen die Einnahme oraler Medikation erschwert, wenn nicht sogar unmöglich sein kann, und für Fälle HIV-assoziierter Pathologie, bei denen schwere Durchfallsymptomatik den Einsatz oraler Medikation unberechenbar macht, bietet sich die parenterale Wirkstoffgabe an. Die geringe Wasserlöslichkeit von Thalidomid (0,012 mg/ml; Arch. Pharm. 321, 371 (1988)) steht jedoch der parenteralen Applikation dieses Wirkstoffes entgegen.

Aus DE 42 11 812 sind Thalidomidderivate bekannt, die am Stickstoffatom des Glutarimidrestes eine Benzoyloxymethylgruppe mit einem eine Aminfunktion tragenden Substituenten besitzen. Diese Thalidomidderivate haben im Vergleich zu Thalidomid eine deutlich höhere Wasserlöslichkeit. Die pH-Werte von wäßrigen Lösungen dieser Verbindungen liegen jedoch deutlich unterhalb des physiologischen pH-Wertbereiches, so daß vor deren Applikation eine Anhebung des pH-Wertes erforderlich ist. Hierbei fallen die entsprechenden Basen aus mit der Folge, daß der Vorteil der höheren Wasserlöslichkeit in erheblichem Umfang oder vollständig aufgehoben wird.

Die der Erfindung zugrundeliegende Aufgabe bestand in der Entwicklung von Thalidomid-Prodrugs, die im physiologischen pH-Wertbereich wasserlöslich sind. Darüber hinaus sollen die zu entwickelnden Verbindungen durch Abspaltung unphysiologischer Prodrug-Reste keine toxikologischen Wirkungen hervorrufen.

Es wurde gefunden, daß die an die zu entwickelnden Verbindungen gestellten Anforderungen von ausgewählten Thalidomid-Prodrugs erfüllt werden.

Gegenstand der Erfindung sind dementsprechend Thalidomid-Prodrugs der Formel I 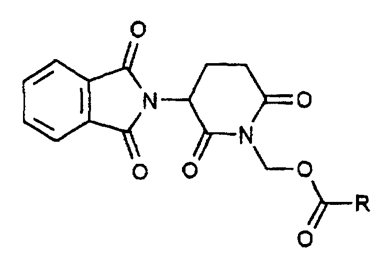 in der R -CHR¹-NHR² oder - (CH₂)ₙCOOH, R¹ H oder C₁₋₄-Alkyl, R² H, C₁₋₃-Alkyl, C(O)-CH₂-NHR³ oder eine Aminoschutzgruppe, R³ H oder eine Aminoschutzgruppe und n eine ganze Zahl zwischen 2 und 4 bedeuten, in Form ihrer Basen oder Salze von physiologischen Säuren.

Die Definition des Restes R¹ C₁₋₄-Alkyl umfaßt sowohl geradkettige als auch verzweigte Kohlenwasserstoffreste, die mit OH, COOH, -C(O)NH₂, NH₂, -NHC(O)NH₂, -NHC(NH)NH₂, S-C₁₋₃-Alkyl oder substituiertem oder unsubstituiertem Phenyl substituiert sein können.

Die Definition von R² C₁₋₃-Alkyl umfaßt geradkettige und verzweigte Kohlenwasserstoffreste.

Als Thalidomid-Prodrugs der Formel I mit R -CHR¹-NHR² eignen sich solche, in denen der Rest R¹ H, CH₃, -CH(CH₃)₂, -CH₂CH(CH₃)₂ oder -CH(CH₃)CH₂CH₃ bedeutet und insbesondere solche Verbindungen, in denen die Reste R¹ H, CH₃ oder -CH(CH₃)₂ und R² H, CH₃, C(O)-OC(CH₃)₃ oder C(O)-O-CH₂-C₆H₅ bedeuten.

Von den Thalidomid-Prodrugs der Formel I, in der R -(CH₂)ₙCOOH bedeutet, eignet sich insbesondere die Verbindung, in der n 2 ist.

Weiterer Erfindungsgegenstand ist ein Verfahren zur Herstellung von Thalidomid-Prodrugs der Formel I mit R -CHR¹-NHR², R¹ H oder C₁₋₄-Alkyl, R² H, C₁₋₃-Alkyl, C(O)-CH₂-NHR³ oder eine Aminoschutzgruppe und R³ H oder eine Aminoschutzgruppe, welches dadurch gekennzeichnet ist, daß N-Hydroxymethylthalidomid mit einer Aminosäure, deren Aminofunktion geschützt ist, in Gegenwart eines Carbodiimids oder Carbonyldiimidazols umgesetzt und anschließend gewünschtenfalls die Schutzgruppe der Aminofunktion acidolytisch abgespalten wird.

Als Schutzgruppe für die Aminofunktion eignen sich insbesondere der t-Butyloxycarbonyl- und der Benzyloxycarbonyl-Rest. Die Umsetzung von N-Hydroxymethylthalidomid mit einer geschützten Aminosäure wird in Gegenwart von äquimolaren bis doppelt-äquimolaren Mengen eines Carbodiimids, beispielsweise Dicyclohexylcarbodiimid, oder eines Carbonyldiimidazols, in einem organischen Lösungsmittel, beispielsweise Dichlormethan, Chloroform, Aceton, Dimethylformamid und/oder Pyridin durchgeführt. Die Umsetzungen können in Gegenwart eines Katalysators, beispielsweise 4-Pyrrolidinopyridin oder 4-Dimethylaminopyridin durchgeführt werden.

Die acidolytische Abspaltung der Aminoschutzgruppe erfolgt vorzugsweise mit Trifluoressigsäure, gegebenenfalls in Gegenwart eines organischen Lösungsmittels, beispielsweise Dichlormethan.

Desweiteren ist Erfindungsgegenstand ein Verfahren zur Herstellung von Thalidomid-Prodrugs der Formel I mit R -(CH₂)ₙCOOH, welches dadurch gekennzeichnet ist, daß N-Hydroxymethylthalidomid mit einem Säureanhydrid in Gegenwart eines Amins umgesetzt wird.

Als Säureanhydrid wird vorzugsweise Bernsteinsäureanhydrid eingesetzt. Als Amine, die bezogen auf N-Hydroxymethylthalidomid üblicherweise in äquimolaren bis doppelt-äquimolaren Mengen eingesetzt werden, eignen sich Triethylamin und/oder Pyridin. Üblicherweise werden die Umsetzungen in einem organischen Lösungsmittel, beispielsweise Dichlormethan, Chloroform, Pyridin und/oder Dimethylformamid in Gegenwart eines Katalysators, beispielsweise 4-Pyrrolidinopyridin oder 4-Dimethylaminopyridin durchgeführt.

Salze erfindungsgemäßer Verbindungen von physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und Asparaginsäure, sind entweder aus den entsprechenden Basen oder den Trifluoracetaten erhältlich. Zur Herstellung der Hydrochloride werden vorzugsweise die entsprechenden Trifluoracetate mit Hilfe eines schwach basischen Anionenaustauschers in die Hydrochloride überführt. Als Lösungsmittel werden kurzkettige aliphatische Alkohole, beispielsweise Methanol, bevorzugt.

Die erfindungsgemäßen Verbindungen sind im physiologischen pH-Wertbereich zwischen 7,0 und 7,5 wasserlöslich applizierbar und toxikologisch unbedenklich. Dementsprechend ist Erfindungsgegenstand auch die Verwendung eines Thalidomid-Prodrugs der Formel I als Wirkstoff in Arzneimitteln, die vorzugsweise parenteral verabreicht werden.

Erfindungsgemäße Arzneimittel enthalten neben mindestens einem Thalidomid-Prodrug der Formel I Trägermaterialien, Füllstoffe, Lösungsmittel, Verdünnungsmittel, Farbstoffe und/oder Bindemittel. Die Auswahl der Hilfsstoffe sowie die einzusetzenden Mengen hängen davon ab, ob das Arzneimittel intravenös, intraperitoneal, intradermal, intramuskulär, intranasal oder lokal appliziert werden soll.

Die an den Patienten zu verabreichende Wirkstoffmenge, die vom Gewicht des Patienten, von der parenteralen Applikationsart, der Indikation und dem Schweregrad der Erkrankung abhängt, liegt üblicherweise zwischen 0,1 und 10 mg/kg eines Thalidomid-Prodrugs der Formel I.

Aufgrund ihrer ausgeprägten immunmodulatorischen Wirkung, die nicht zu einer generellen Immunsuppression führt, eignen sich Thalidomid-Prodrugs der Formel I zur Behandlung aller Erkrankungen, die durch hohe TNF-α-Bildung oder fokale Vaskuliden charakterisiert sind.

### Beispiele

### Herstellung erfindungsgemäßer Verbindungen

t-Butyloxycarbonyl-geschützte Aminosäuren wurden nach der in Hoppe-Seyler's Z. physiol. Chem. 357, 1651 (1976) beschriebenen Methode hergestellt.

Säulenchromatographische Trennungen wurden mit Kieselgel der Korngröße 0,05 - 0,2 mm der Firma Merck durchgeführt.

Die Wasserlöslichkeit wurde UV-spektroskopisch bei 300 nm und 25° C bestimmt.

### Beispiel 1

### N-t-Butyloxycarbonyl-2-aminoessigsäure-[3-(1,3-dihydro-1,3-dioxo-2H-isoindol-2-yl)-2,6-dioxopiperidin-1-yl]-methylester (1)

2,88 g (10 mmol) N-Hydroxymethylthalidomid, 1,75 g (10 mmol) N-t-Butyloxycarbonyl-glycin, 2,06 g (10 mmol) Dicyclohexylcarbodiimid und 0,15 g (1 mmol) 4-Pyrrolidinopyridin wurden in 50 ml trockenem Dichlormethan 24 Stunden bei Raumtemperatur gerührt. Anschließend wurde der ausgefallene Dicyclohexylharnstoff abfiltriert und das Filtrat zuerst mit Essigsäure und dann mit Wasser ausgeschüttelt. Nach dem Trocknen der organischen Phasen mit Magnesiumsulfat und der destillativen Entfernung des Lösungsmittels wurde der Rückstand aus Ethanol umkristallisiert. Es wurden 2,59 g (58 % der Theorie) Verbindung 1 mit einem Schmelzpunkt von 108 bis 111° C erhalten.

### Beispiel 2

### 2-Aminoessigsäure-[3-(1,3-dihydro-1,3-dioxo-2H-isoindol-2-yl)-2,6-dioxo-piperidin-1-yl]methylester, Hydrochlorid (2)

1,78 g (4 mmol) der nach Beispiel 1 hergestellten Verbindung 1 wurden in 20 ml einer Mischung aus 25 Volumen-% Trifluoressigsäure und 75 Volumen-% Dichlormethan eine Stunde bei Raumtemperatur gerührt. Anschließend wurde das Lösungsmittel im Vakuum entfernt und der erhaltene Ruckstand mit Dichlormethan koevaporiert. Das erhaltene Trifluoracetat wurde in 160 ml Methanol gelöst und mittels Ionenaustauschchromatographie in das Hydrochlorid überführt (als Ionenaustauscher wurde Amberlite IR 45 verwendet, der zuvor mit 1N Salzsäure in die Cl⁻-Form überführt worden war). Nach destillativer Entfernung des Lösungsmittels wurde der erhaltene Rückstand in siedendem Ethanol suspendiert und tropfenweise mit Wasser bis zur Bildung einer klaren Lösung versetzt. Es wurden 0,81 g (53 % der Theorie) Verbindung 2 mit einem Schmelzpunkt von 227 bis 232° C und einer Wasserlöslichkeit von 49,7 mg/ml entsprechend 33,6 mg/ ml Thalidomid erhalten.

### Beispiel 3

### N-t-Butyloxycarbonyl-2-methylaminopropionsäure-[3-(1,3-dihydro-1,3-dioxo-2H-isoindol-2-yl)-2,6-dioxo-piperidin-1-yl]methylester (3)

4,32 g (15 mmol) N-Hydroxymethylthalidomid, 3,05 g (15 mmol) N-t-Butyloxycarbonyl-L-N-methylalanin, 3,09 g (15 mmol) Dicyclohexylcarbodiimid und 0,22 g (1,5 mmol) 4-Pyrrolidinopyridin wurden in 75 ml trockenem Dichlormethan 24 Stunden bei Raumtemperatur gerührt. Der ausgefallene Dicyclohexylharnstoff wurde abfiltriert und das Filtrat zuerst mit Essigsäure und dann *mit* Wasser ausgeschüttelt. Nach dem Trocknen der organischen Phasen mit Magnesiumsulfat, destillativer Entfernung des Lösungsmittels und säulenchromatographischer Reinigung des Rückstandes mit Dichlormethan/Aceton im Volumenverhältnis 9 : 1 wurden 4,44 g (63 % der Theorie) Verbindung 3 mit einem Schmelzpunkt von 123 bis 125° C erhalten.

### Beispiel 4

### 2-Methylaminopropionsäure-[3-(1,3-dihydro-1,3-dioxo-2H-isoindol-2-yl)-2,6-dioxopiperidin-1-yl]methylester, Hydrochlorid (4)

Die nach Beispiel 3 erhaltene Verbindung 3 wurde unter den in Beispiel 2 angegebenen Bedingungen in Verbindung 4 überführt. Durch Kristallisation aus Methanol/Diethylether wurden 1,05 g (64 % der Theorie) Verbindung 4 mit einem Schmelzpunkt von 147 bis 151° C und einer Wasserlöslichkeit von >300 mg/ml, entsprechend >190 mg/ml Thalidomid erhalten.

### Beispiel 5

### N-t-Butyloxycarbonyl-2-amino-3-methylbuttersäure-[3-(1,3-dihydro-1,3-dioxo-2H-isoindol-2-yl)-2,6-dioxopiperidin-1-yl]methylester (5)

Unter den in Beispiel 3 angebenen Bedingungen wurden aus 4,32 g (15 mmol) N-Hydroxymethylthalidomid, 3,26 g (15 mmol) N-t-Butyloxycarbonyl-L-valin, 3,09 g (15 mmol) Dicyclohexylcarbodiimid und 0,22 g (1,5 mmol) 4-Pyrrolidinopyridin 3,85 g (53 % der Theorie) Verbindung 5 mit einem Schmelzpunkt von 82 bis 85° C erhalten.

### Beispiel 6

### 2-Amino-3-methylbuttersäure-[3-(1,3-dihydro-1,3-dioxo-2H-isoindol-2-yl)-2,6-dioxopiperidin-1-yl]methylester, Hydrochlorid (6)

1,95 g (4 mmol) der nach Beispiel 5 erhaltenen Verbindung 5 wurden unter den in Beispiel 2 angegebenen Bedingungen in Verbindung 6 überführt. Die Kristallisation aus Ethanol/Diethyiether ergab 1,01 g (60 % der Theorie) Verbindung 6 mit einem Schmelzpunkt von 145 bis 150° C und einer Wasserlöslichkeit von >300 mg/ml entsprechend >190 mg/ml Thalidomid.

### Beispiel 7

### 2-(N-t-Butyloxycarbonyl-aminomethylcarbonylamino)essigsäure-[3-(1,3-dihydro-1,3-dioxo-2H-isoindol-2-yl)-2,6-dioxopiperidin-1-yl]-methylester (7)

Unter den in Beispiel 1 angebenen Bedingungen wurden aus 2,88 g (10 mmol) N-Hydroxymethylthalidomid, 2,32 g (10 mmol) N-t-Butyloxycarbonylglycylglycin, 2,06 g (10 mmol) Dicyclohexylcarbodiimid und 0,15 g (1 mmol) 4-Pyrrolidinopyridin 3,67 g (73 % der Theorie) Verbindung 7 mit einem Schmelzpunkt von 178 bis 181° C erhalten.

### Beispiel 8

### 2-(Aminomethylcarbonylamino)essigsaure-[3-(1,3-dihydro-1,3-dioxo-2H-isoindol-2-yl)-2,6-dioxo-piperidin-1-yl]-methylester, Hydrochlorid (8)

Aus 2,01 g (4 mmol) der nach Beispiel 7 hergestellten Verbindung 7 wurden unter den in Beispiel 2 angegebenen Bedingungen 1,35 g (77 % der Theorie) Verbindung 8 mit einer Wasserlöslichkeit von >300 mg/ml, entsprechend >190 mg/ml Thalidomid erhalten.

### Beispiel 9

### [3-(1,3-Dihydro-1,3-dioxo-2H-isoindol-2-yl)-2,6-dioxopiperidin-1-yl]methoxycarbonylpropionsäure (9)

2,88 g (10 mmol) N-Hydroxymethylthalidomid, 2 g (20 mmol) Bernsteinsäureanhydrid, 2,8 ml (20 mmol) Triethylamin und 0,15 g (1 mmol) 4-Pyrrolidinopyridin wurden in 50 ml trockenem Dichlormethan 24 Stunden bei Raumtemperatur gerührt. Anschließend wurde das Reaktionsgemisch zuerst mit 5 %iger Salzsäure und anschließend mit Wasser ausgeschüttelt. Nach Trocknen der organischen Phasen über Magnesiumsulfat und destillativer Entfernung des Lösungsmittels wurde der erhaltene Rückstand mit einer geringen Menge an Ethylacetat versetzt und die ausgefallenen Kristalle aus Ethanol umkristallisiert. Es wurden 2,66 g (68 % der Theorie) Verbindung 9 mit einem Schmelzpunkt von 143 bis 146° C und einer Wasserlöslichkeit von 16,7 mg/ml, entsprechend 11,1 mg/ml Thalidomid erhalten.

### Pharmakologische Untersuchungen

### Wirksamkeit erfindungsgemäßer Verbindungen in vitro

Die Freisetzung von TNF-α wurde in vitro an humanen mononukleären Zellen des peripheren Blutes (T-Zellen, B-Zellen und Monozyten) nach Stimulation mit Lipopolysaccharid (LPS) untersucht. LPS ist ein Bestandteil der bakteriellen Zellwand und stimuliert Monozyten und Makrophagen.

Mononukleäre Zellen wurden aus dem heparinisierten Blut von mindestens drei freiwilligen Spendern gewonnen. Hierzu wurden je 20 ml Blut in bekannter Weise über einen Ficoll-Paque-Gradienten getrennt. Die Zellen wurden geerntet und dreimal mit einem Zellkulturmedium gewaschen. Das verwendete Zellkulturmedium bestand aus RPMI 1640 Medium supplementiert mit 2 mM Glutamin (Life Technologies, Eggenstein), 10 % fötalem Kälberserum (Life Technologies), 50 µg/ml Streptomycin (Sigma, Deisenhofen), 50 IU/ml Penicillin (Sigma) und 100 µM β-Mercaptoethanol (Merck, Darmstadt). Die mononukleären Zellen wurden anschließend in 15 ml Zellkulturmedium aufgenommen und in 1 ml Ansätzen in sterilen 24-Loch-Inkubationsplatten (Sigma) aufgeteilt. Den 1-ml-Ansätzen, die man als Kontrollansatz benutzte, wurden jeweils 1 µl Dimethylsulfoxid (DMSO, Merck) zugesetzt. Den Testansätzen wurde 1 µl einer Lösung einer erfindungsgemäßen Verbindung (in DMSO; Endkonzentrationen im Test: 0,5; 5; 12,5 und 50 µg/ml) zugegeben. Die Ansätze wurden eine Stunde im CO₂-Brutschrank (5 % CO₂, 90 % Luftfeuchtigkeit) inkubiert. Anschließend wurden, mit Ausnahme der Kontrollansätze, jeweils 2,5 µg LPS (von E. coli 0127:B8; Sigma, Deisenhofen) als Stimulans zugefügt. Die Inkubation der Ansätze wurde 20 Stunden fortgesetzt. Die Konzentration von TNF-α in den Zellkulturüberständen der Ansätze wurde im Anschluß an die Inkubation mit ELISA-Tests (Boehringer-Mannheim) bestimmt. Aus den Meßwerten der Kontrollansätze und den mit den erfindungsgemäßen Verbindungen inkubierten Testansätzen wurde die Stärke der Hemmung der TNF-α Freisetzung bestimmt. Mit Hilfe einer Regressionsgerade wurden die Konzentrationen errechnet, die zu einer 50 %igen Hemmung der TNF-α Freisetzung führten (IC₅₀-Werte).

In der nachfolgenden Tabelle ist der inhibitorische Einfluß der erfindungsgemäßen Verbindungen auf die LPS-induzierte Freisetzung von TNF-α dargestellt:

| erfindungsgemäße Verbindung | Hemmung der TNF-α-Freisetzung bei einer Endkonzentration von 50 µg/ml | IC₅₀ [µg/ml] |
|---|---|---|
| 1 | 78 % | 2,7 |
| 2 | 54 % | |
| 6 | 97 % | 2,0 |
| 9 | 35 % | |

### Wirksamkeit erfindungsgemäßer Verbindungen im Tiermodell

Zur in vivo-Charakterisierung der immunpharmakologischen Wirkungen erfindungsgemäßer Verbindungen wurde ein Testmodell gewählt, in dem T-Lymphozyten stimuliert wurden. Die Relevanz des immunpharmakologischen Tiermodells ergibt sich aus der erforderlichen Zell-Zell-Kooperation bei der Einleitung einer Immunantwort. Durch die Wechselwirkung zwischen antigenpräsentierenden Zellen, z. B. Monozyten, mit T-Zellen wird erst die Voraussetzung für eine Aktivierung und klonale Expansion der T-Zellen geschaffen. Diese ist Charakteristikum sowohl von Infektionsabwehr als auch von Autoimmunaggression oder Transplantatabstoßung oder Transplantat-gegen-Wirtsreaktion (Graft-vs. Host-Disease). Lymphozyteninfiltrate stellen das Initalstadium von zum Beispiel chronischen Graft-vs.Host-Reaktionen, aber auch von akuten aphthösen Läsionen in der Mundschleimhaut dar, wie sie bei Morbus Behcet oder als sogenannte idiopathische Aphthen auftreten.

Die Stimulation von T-Zellen wurde durch intravenöse Applikation des Staphylokkoken Enterotoxin B (SEB; 200 µg) an Galaktosamin-vorbehandelten balb/c-Mäusen erreicht. SEB ist ein Superantigen, das einerseits MHC-Moleküle auf antigenpräsentierenden Zellen, andererseits invariable Strukturen auf bestimmten T-Zell-Rezeptorfamilien bindet. Hierbei kommt es zur Aktivierung sowohl der T-Zelle als auch der Monozyten. Als Parameter für die T-Zell-Aktivierung wurde die Konzentration des Zytokins Interleukin-2 (IL-2) im Serum mittels eines kommerziellen ELISA-Tests, der spezifisch murines IL-2 detektiert, bestimmt. Die Injektion von SEB führte zu einem zeitabhängigen Anstieg der Serum-IL-2-Spiegel mit einem deutlichen Maximum zwei Stunden nach SEB-Applikation. Die Herkunft von im Serum gemessenen IL-2 aus T-Zellen konnte verifiziert werden, indem SEB T-Zell-defizienten SCID-Mäusen appliziert wurde, die daraufhin kein IL-2 bildeten.

Die erfindungsgemäßen Verbindungen wurden in 1 %iger wäßriger Carboxymethylcellulose (CMC) gelöst und den Tieren in Dosierungen von 100 bis 400 mg/kg und Volumina von 1 ml 30 Minuten vor der SEB-Applikation intraperitoneal appliziert. Die Tiere der Kontrollgruppe erhielten 1 ml/kg 1 %ige wäßrige CMC-Lösung intraperitoneal appliziert. Die Serum-IL-2-Konzentrationen wurden 2 Stunden nach Applikation von SEB bestimmt. In der nachfolgenden Tabelle sind die maximalen Hemmeffekte (in %) der Serum-IL-2-Spiegel in den mit einer erfindungsgemäßen Verbindung behandelten Gruppen im Vergleich zur Kontrollgruppe angegeben. Die Prozentangaben stellen Mittelwerte von jeweils 6 bis 8 Einzelversuchen dar. Über eine Regressionsgerade wurden die Dosierungen errechnet, die zu einer 40 % igen Reduktion der Serum-IL-2-Konzentrationen führten (ED₄₀-Werte).

| erfindungsgemäße Verbindung | Max. Hemmung des Serum-IL-2-Anstieges bei einer Dosis von 400 mg/kg | ED₄₀ [mg/kg] |
|---|---|---|
| 1 | 34 % | |
| 2 | 68 % | 230 |
| 4 | 26 % | |
| 6 | 52 % | |

Vergleichende Untersuchungen zeigten, daß die erfindungsgemäßen Verbindungen im Gegensatz zu Glukokortikoiden den Anstieg des Serum-IL-2 auch dann hemmten, wenn sie 30 Minuten vor der Stimulation durch SEB verabreicht wurden.Für eine hemmende Wirkung durch Glukokortikoide war es dagegen erforderlich, daß diese Substanzen 18 Stunden vor der Stimulation durch SEB appliziert wurden.

## Patentansprüche

1. Thalidomid-Prodrugs der Formel I 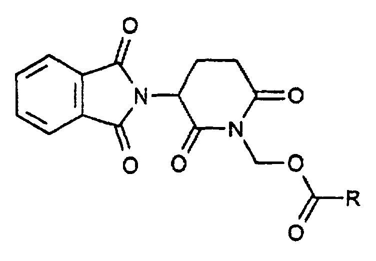 in der R -CHR¹-NHR² oder -(CH₂)ₙCOOH bedeutet, R¹ H oder C₁₋₄-Alkyl, R² H, C₁₋₃-Alkyl, C(O)-CH₂-NHR³ oder eine Aminoschutzgruppe, R³ H oder eine Aminoschutzgruppe und n eine ganze Zahl zwischen 2 und 4 bedeuten, in Form ihrer Basen oder Salze von physiologischen Säuren.

2. Thalidomid-Prodrugs gemäß Anspruch 1, **dadurch gekennzeichnet, daß** R¹ H, CH₃, -CH(CH₃)₂, -CH₂CH(CH₃)₂ oder -CH(CH₃)CH₂CH₃ bedeutet.

3. Thalidomid-Prodrugs gemäß einem oder beiden der Ansprüche 1 bis 2, **dadurch gekennzeichnet, daß** R¹ H, CH₃ oder -CH(CH₃)₂ und R² H, CH₃, C(O)-OC(CH₃)₃ oder C(O)-O-CH₂-C₆H₅ darstellen.

4. Thalidomid-Prodrugs nach Anspruch 1, **dadurch gekennzeichnet, daß** n 2 bedeutet.

5. Verfahren zur Herstellung von Thalidomid-Prodrugs der Formel I gemäß Anspruch 1, in der R -CHR¹-NHR² bedeutet, **dadurch gekennzeichnet, daß** man N-Hydroxymethylthalidomid mit einer Aminosaure, deren Aminofunktion geschützt ist, in Gegenwart eines Carbodiimids oder eines Carbonyldiimidazols umsetzt und anschließend gewünschtenfalls die Schutzgruppe der Aminofunktion acidolytisch abspaltet.

6. Verfahren nach Anspruch 5, **dadurch gekennzeichnet, daß** man eine Aminosäure einsetzt, deren Aminofunktion durch die t-Butyloxycarbonyl- oder die Benzyloxycarbonylgruppe geschützt ist.

7. Verfahren nach einem oder mehreren der Ansprüche 5 bis 6, **dadurch gekennzeichnet, daß** man die acidolytische Abspaltung mit Trifluoressigsäure durchführt.

8. Verfahren zur Herstellung eines Thalidomid-Prodrugs der Formel I gemäß Anspruch 1, in der R -(CH₂)ₙCOOH bedeutet, **dadurch gekennzeichnet, daß** man N-Hydroxymethylthalidomid mit einem Säureanhydrid in Gegenwart eines Amins umsetzt.

9. Verwendung eines Thalidomid-Prodrugs nach Anspruch 1 als Wirkstoff in einem Arzneimittel.

10. Verwendung nach Anspruch 9, **dadurch gekennzeichnet, daß** das Arzneimittel parenteral appliziert wird.

## Claims

1. Thalidomide prodrugs of the formula I 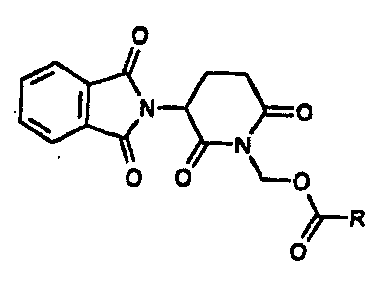 in which R denotes -CHR¹-NHR² or -(CH₂)ₙCOOH, R¹ denotes H or C₁₋₄-alkyl, R² denotes H, C₁₋₃-alkyl, C(O)-CH₂-NHR³ or an amino-protective group, R³ denotes H or an amino-protective group and n denotes an integer between 2 and 4, in the form of their bases or salts of physiological acids.

2. Thalidomide prodrugs according to claim 1, **characterized in that** R¹ denotes H, CH₃, -CH(CH₃)₂, -CH₂CH(CH₃)₂ or -CH(CH₃)CH₂CH₃.

3. Thalidomide prodrugs according to one or both of claims 1 to 2, **characterized in that** R¹ represents H, CH₃ or -CH(CH₃)₂ and R² represents H, CH₃, C(O)-OC(CH₃)₃ or C(O)-O-CH₂-C₆H₅.

4. Thalidomide prodrugs according to claim 1, **characterized in that** n denotes 2.

5. Process for the preparation of thalidomide prodrugs of the formula I according to claim 1 in which R denotes -CHR¹-NHR², **characterized in that** N-hydroxymethylthalidomide is reacted with an amino acid, the amino function of which is protected, in the presence of a carbodiimide or a carbonyldiimidazole and, if desired, the protective group of the amino function is then split off by acidolysis.

6. Process according to claim 5, **characterized in that** an amino acid in which the amino function is protected by the t-butyloxycarbonyl or the benzyloxycarbonyl group is employed.

7. Process according to one or more of claims 5 to 6, **characterized in that** the splitting off by acidolysis is carried out with trifluoroacetic acid.

8. Process for the preparation of a thalidomide prodrug of the formula I according to claim 1 in which R denotes -(CH₂)ₙCOOH, **characterized in that** N-hydroxymethylthalidomide is reacted with an acid anhydride in the presence of an amine.

9. Use of a thalidomide prodrug according to claim 1 as an active compound in a medicament.

10. Use according to claim 9, **characterized in that** the medicament is administered parenterally.

## Revendications

1. Promédicaments du thalidomide, de formule I : 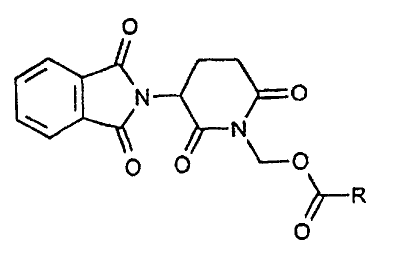 dans laquelle R représente un groupe -CHR¹-NHR² ou -(CH₂)ₙCOOH, R¹ représente H ou un groupe alkyle en C₁ à C₄, R² représente H, un groupe alkyle en C₁ à C₃, C(O)-CH₂-NHR³ ou un groupe protégeant la fonction amino, R³ représente H ou un groupe protégeant la fonction amino et n est un nombre entier entre 2 et 4, sous forme de leurs bases ou de leurs sels d'acides acceptables du point de vue physiologique.

2. Promédicaments du thalidomide suivant la revendication 1, **caractérisés en ce que** R¹ représente H, CH₃, -CH(CH₃)₂, -CH₂CH(CH₃)₂ ou -CH (CH₃) CH₂CH₃.

3. Promédicaments du thalidomide suivant l'une ou l'autre des revendications 1 et 2 ou les deux, **caractérisés en ce que** R¹ représente H, CH₃ ou -CH(CH₃)₂ et R² représente H, CH₃, C (O)-OC (CH₃)₃ ou C (O)-O-CH₂-C₆H₅.

4. Promédicaments du thalidomide suivant la revendication 1, **caractérisés en ce que** n est égal à 2.

5. Procédé de production de promédicaments du thalidomide de formule I suivant la revendication 1, dans lequel R est un groupe -CHR¹-NHR², **caractérisé en ce que** l'on fait réagir le N-hydroxyméthylthalidomide avec un amino-acide dont la fonction amino est protégée, en présence d'un carbodiimide ou d'un carbonyldiimidazole, puis on élimine si nécessaire par acidolyse le groupe protecteur de la fonction amino.

6. Procédé suivant la revendication 5, **caractérisé en ce que** l'on utilise un amino-acide dont la fonction amino est protégée par le groupe tertiobutyloxycarbonyle ou par le groupe benzyloxycarbonyle.

7. Procédé suivant l'une ou l'autre des revendications 5 et 6, ou les deux, **caractérisé en ce que** l'on conduit l'élimination par acidolyse avec l'acide trifluoracétique.

8. Procédé de production d'un promédicament du thalidomide de formule I suivant la revendication 1, dans laquelle R est un groupe -(CH₂)ₙCOOH, **caractérisé en ce que** l'on fait réagir le N-hydroxyméthylthalidomide avec un anhydride d'acide en présence d'une amine.

9. Utilisation d'un promédicament du thalidomide suivant la revendication 1 comme substance active dans un médicament.

10. Utilisation suivant la revendication 9, **caractérisée en ce que** le médicament est administré par voie parentérale.
